# EUROPEAN PATENT APPLICATION

(11) **EP 2 233 142 A1**
(43) Date of publication of application: **29.09.2010**
(21) Application number: 08860823.7
(22) Date of filing: 09.12.2008
(51) Int. Cl.: A61K 31/663, A61K 9/70, A61K 47/10, A61K 47/28, A61K 47/32, A61P 19/08, A61P 19/10

(54) **TRANSDERMALLY ABSORPTIVE PREPARATION**

(30) Priority: 10.12.2007 JP 2007341687; 31.01.2008 JP 2008046818; 22.10.2008 JP 2008293889
(71) Applicant: Cosmed Pharmaceutical Co., Ltd., Kamigyo-ku Kyoto-shi Kyoto 602-0841 (JP)
(72) Inventor: YAMAMOTO, Akira, Kyoto-shi Kyoto 607-8414 (JP); SAKANE, Toshiyasu, Kyoto-shi Kyoto 607-8414 (JP); KATSUMI, Hidemasa, Kyoto-shi Kyoto 607-8414 (JP); KAMIYAMA, Fumio, Kyoto-shi Kyoto 602-0841 (JP); QUAN, Ying-shu, Kyoto-shi Kyoto 602-0841 (JP)
(74) Representative: Merkle, Gebhard
(86) International application number: PCT/JP2008/072318
(87) International publication number: WO 2009/075258

(57) **Abstract**

Summary

This invention is to provide a transdermal administration type pharmaceutical preparation which has better transdermal absorptivity, safty and practical application for treatment of bone calcium metabolic diseases, such as high calcium in blood which resulted from osteoporosis, osteitis deformans and malignant tumors.

A percutaneous administration type pharmaceutical preparation in tape form comprising a flexible backing, which is not permeable by the active ingredient, and an adhesive base layer formed on said flexible backing; said adhesive base layer consisting essentially of an adhesive base material of acrylic copolymer, polyhydric alcohol and bisphosphonate or its derivatives, wherein the bisphosphonate or its derivatives are in their therapeutic dose, adhesive base material and polyhydric alcohol in a concenrtration ratio of 100 : 150 to 600 by weight; said acrylic copolymer are copolymerized by acrylic acid, 2-ethylhexyl acrylate and polar monomer (except for acrylic acid) in the concentration ratio of 50 to 88% : 8 to 40% : 1 to 40% by weight respectively. PEG modify and antioxidants are also applicable to reduce the skin stimulation.

## Description

### Background of the invention

### 1. Field of invention

The present invention relates to a transdermal administration type pharmaceutical preparation for treatment of bone calcium metabolic disease.

### 2. Description of the prior art

Oral preparation or injection containing bisphosphonate has been used for treatment of bone calcium metabolic diseases, such as high calcium in blood which resulted from osteoporosis, osteitis deformans and malignant tumors (patent 1).

However, the absorptivity of bisphosphonate by oral medication was quite low as 1 to 2 %, and even an 80 to 90% decrease of the absorptivity can be observed when medication together with meal. Besides, bisphosphonate has strong stimulation against mucous membrane of stomach and esophagus, and a horizontal position should be avoided within 30 minutes after taking medication for the protection of mucous membrane from injury, resulting in a big burden for 70% of those osteoporosis patients who are elderly at old age. As for injection, it is not only time and money consuming for injection acceptance from nurses and physicians, but also to cause bacterial infection if not sterilized before injection. Besides, it is possible to cause acute high blood concentration of bisphosphonate after injection.

Although it is expected to develop a bisphosphonate pharmaceutical preparation which is not necessarily to avoid a horizontal position within 30 minutes after taking medication and is suitable for elderly patient lying in bed, transdermal preparations in lotion, ointment, cream, cataplasm and tape forms have not been developed (patents 2-4).
[patent 1] No. 2000-517324
[patent 2] No. 2004-250330
[patent 3] No. 2004-250423
[patent 4] No. 2006-213658

The present invent relates to transdermal administration type pharmaceutical preparations in lotion, ointment, cream, cataplasm and tape forms containing bisphosphonate as an active ingredient therein for treatment of bone calcium metabolic disease.

### Description of the invention

An object of this invention is to overcome the said deficiency of the oral preparation and injection, to provide a transdermal administration type pharmaceutical preparation which has better transdermal absorptivity, safty and practical application for treatment of bone calcium metabolic diseases, such as high calcium in blood which resulted from osteoporosis, osteitis deformans and Malignant tumors

The transdermal administration type pharmaceutical preparation in tape form of this invention comprises of a flexible backing, and an acrylic adhesive base layer essentially containing bisphosphonate or its derivatives formed on said flexible backing.

The said bisphosphonate, for example, alendronate, incadronate, etidronate, olpadronate, clodronate, zoledronate, tiludronate, neridronate, pamidronate and risedronate, has been used to treat high calcium in blood which is caused by bone calcium metabolic diseases, such as osteoporosis, osteitis deformans and malignant tumors.

The said bisphosphonate may be the derivatives which are synthesized by the reaction between amino group of bisphosphonate and polyethyleneglycol (PEG), amino acid, or carboxylic acid etc. Transdermal absorptivity of these derivatives is slightly lower than that of bisphosphonate itself, but skin stimulation is reduced remarkably and practical application is improved.

The said bisphosphonate includes its derivatives which were synthesized by the reaction between amino group of bisphosphonate and polyethyleneglycol (PEG), amino acid, or carboxylic acid etc.

The synthesis method of bisphosphonate derivatives of the transdermal administration type pharmaceutical preparation in tape form according to this invention is not specifically limited.

The said derivatives can be synthesized by any known methods, such as the following way:
(1) Polyetyleneglycol (PEG)-modified alendronate is synthesized by the reaction between methoxypolyethyleneglycol-N-succinimidylsuccinate which is polyetyleneglycol activated against amino group, and amino group of alendronate.
(2) N-t-Boc amino acid-N-hydroxysuccinimide ester is synthesized by the reaction between N-t-Boc amino acid and N-hydroxysuccinimide. Subsequently, N-t-Boc amino acid-N-hydroxysuccinimide ester is reacted with the amino group of alendronate, N-t-Boc amino acid- alendronate is obtained. By deprotecting t-Boc group, amino acid- alendronate is obtained.
(3) Fatty acid- N-hydroxysuccinimide ester is synthesized by the reaction between fatty acid and N-hydroxysuccinimide. Subsequently, fatty acid- N-hydroxysuccinimide ester is reacted with the amino group of alendronate to obtain the fatty acid - alendronate.

The said transdermal administration preparation can be in any known topical forms of tape, patch, cataplasm, ointment, cream, lotion, mouth preparations, eye drops or suppository. It is more preferably in tape form.

The said transdermal administration preparation in forms of tape, patch and cataplasm etc. in this invention consists a base layer formed on the flexible backing; said adhesive base layer consisting essentially of a base material and bisphosphonate or its derivatives compatible with said base material.

The said flexible backing in this invention can be a single film of any known material used in preparations of tape, patch and cataplasm etc., such as cellulose acetate, ethyl cellulose, polyethylene, polypropylene, ethylene-propylene copolymer, ethylene-vinyl acetate copolymer, polyvinyl chloride, vinylidene chloride, vinyl acetate-polyvinyl chloride copolymer, polyamide, polyester, ABS resin, SIS resin, SEBS resin, urethane resin, silicon resin, aluminum etc. Besides, the said flexible backing could be a single woven or non woven; or a laminated woven or non woven.

The said base material in this invention can be any material used in transdermal preparations, for example the adhesive being used in transdermal preparations in patch and cataplasm forms. Since a better adhesiveness to skin and no residual of the adhesive on skin is necessary for transdermal preparation in tape form, it is better to use acrylic adhesive or rubber adhesive. On the other hand, a base material composed of water and water soluble resin is used for transdermal preparation in patch form.

0018 The said acrylic adhesive in this invention is preferably a copolymer of fatty alcohols which have 4 to 18 numbers of carbon atom and alkyl methacrylate ester, or a copolymer of alkyl methacrylate ester and functional monomer.

0019 The examples of said alkyl methacrylate ester in this invention are butyl methacrylate, isobutyl methacrylate, hexyl methacrylate, octyl methacrylate, 2-ethylhexyl methylacrylate, isooctyl methacrylate, isodecyl methacrylate, lauryl methacrylate, stearyl methacrylate etc.

0020 The examples of said functional monomers in this invention are 2-hydroxyethyl methacrylate, hydroxypropyl methacrylate, methacrylic acid, Maleic acid, maleic anhydride, Fumaric Acid, crotonic acid, butyl maleic acid, acrylamide, dimethylamide, diethyl acrylamide, butoxymethyl acrylamide, ethoxymethyl, methylol (meth)acrylamide, dimethylamino acrylate, N-Vinyl-2-pyrrolidone etc.

0021 The said alkyl methacrylate ester in this invention may copolymerize with reactive monomer. Examples of reactive monomers are vinyl acetate, styrene, α-methyl styrene, vinyl chloride, acrylonitrile, ethylene, propylene, butadiene. More specifically, the copolymer contains alkyl methacrylate ester in a concentration of more than 50% by weight of the total amount of monomers.

0022 Since bisphosphonate and derivatives are water soluble, it is desired to contain more functional monomer of acrylic acid in weight in the adhesive for a better solubility of bisphosphonate and derivatives in it and prevention from the crystallization of bisphosphonate and derivatives after a prolonged time. The said adhesive that containing more acrylic acid is preferable. However, more addition of acrylic acid can lead to hardness of the adhesive which may result in a lower adhesion of the adhesive. It is preferable that the adhesive comprises a copolymer and polyhydric alcohol in the concentration ratio of 100 : 150 to 600 by weight. The said copolymer comprises acrylic acid, 2-ethylhexyl acrylate and polar monomer (except for acrylic acid) in the concentration ratio of 50 to 80% : 8 to 40% : 1 to 42% by weight.

0023 If the concentration ratio of acrylic acid in said acrylic copolymer is less than 50% by weight, the lipophilicity of the adhesive will be enhanced and the adhesion of the adhesive to skin will be reduced. If the concentration ratio of acrylic acid in said acrylic copolymer is more than 80% by weight, the hydrophlicity will be enhanced and the adhesion of the adhesive to wet skin will be reduced. It is more preferable acrylic acid is in a concentration ratio of 70 to 85% by weight.

0024 If the concentration ratio of 2-ethylhexyl acrylate in said acrylic copolymer is less than 8% by weight, the skin adhesion of the adhesive will be lower. If the concentration ratio of 2-ethylhexyl acrylate is more than 40% by weight, the lipophilicity of the adhesive will be enhanced and the solubility of bisphosphonate and its derivatives will be reduced. It is more preferable in a concentration ratio of 70 to 85% by weight. It is more preferable 2-ethylhexyl acrylate is in a concentration ratio of 8 to 40% by weight.

0025 If the concentration ratio of polar monomer (except for acrylic acid) in said acrylic copolymer is less than 1% by weight, the cohesive force of the copolymer will be reduced and it is difficult to prevent from skin remaining of the adhesive when the patch to be peeled off the skin. If the concentration ratio of polar monomer is more than 42% by weight, the skin adhesion of the adhesive will be reduced. To obtain a suitable adhesiveness of the adhesive, it is preferable the polar monomer is in a concentration ratio of 1 to 42% by weight.

0026 Examples of said polar monomer are N-vinyl-2-pyrollidone, 2-hydroxyethyl acrylate, vinyl acetate, methoxyethyl acrylate, acrylamide etc. It is more preferable to select at lease one from N-vinyl-2-pyrollidone, vinyl acetate, methoxyethyl acrylate, acrylamide and 2-hydroxyethyl acrylate.

0027 If the 2-hydroxyethyl acrylate in said acrylic acid is substituted by alkyl acrylate ester which has a short alkyl chain, for example butyl acrylic acid, the lipophilicity of the copolymer will be not sufficient and the adhesion of the adhesive to dry skin will be lower. However, if the alkyl acrylate ester which has a long alkyl chain is used, the lipophilicity of the copolymer will be too strong to form a homogeneous solution with acrylic acid. Therefore, it is preferable the copolymer is copolymerized from the said components in the said concentration ratio.

0028 The said adhesive base material can be polymerized by any known methods, such as water polymerization, solution polymerization, suspension polymerization etc. The monomers for copolymerization consists more acrylic acid by weight. Because it is desired that water soluble bisphosphonate and it derivatives can be dissolved homogeneously in adhesive, it is preferable to select a solvent which can dissolve acrylic acid, 2-hydroxyethyl acrylate and polar monomer into a homogeneous solution. Therefore, it is preferable to use a mixed solvent of water-acetone to dissolve acrylic acid, 2-hydroxyethyl acrylate and polar monomer into a homogeneous solution.

0029 The said acrylic copolymer will lose its well adhesiveness if it is too hard. In order to increase the adhesiveness of the adhesive, it is necessary to add plasticizer of polyhydric alcohol into the copolymer in a concentration ratio of 150 to 600:100 by weight. The addition of polyhydric alcohol can help us to obtain an adhesive which has not only better adhesion to both wet and dry skin, but also sufficient adhesive force. If the polyhydric alcohol is added less than 150 by weight, its plasticity for adhesive will be small and the adhesion of the adhesive will be too weak. If the polyhydric alcohol is added more than 600 by weight, the copolymer will be over plasticized and the balance between cohesive force and adhesion can not be well reached.

0030 The examples of the said polyhydric alcohol are glycerin, diglycerin, propylene glycol, butylenes glycol, polyethylene glycol and polypropylene glycol etc. They can be used alone or mixed.

0031 0031

It is desired that a metal chelate is added into the said acrylic copolymer for cross-linking to obtain a moderate cohesive force and adhesion. It is preferable that acrylic copolymer and metal chelate are in a concentration ratio of 100:0.05 to 2.0 by weight.

0032 Examples of said metal chelates are aluminum acetylacetonate, aluminium hydrate gel and aluminium glycinate. They can be used alone or combined.

0033 The said rubber adhesives are those known rubber adhesives, for example, natural rubber, isoprene, polyisobutylene, polybutadiene, styrene-butadiene copolymer, styrene-butadiene-styrene copolymer, styrene-isoprene copolymer, styrene- isoprene-styrene copolymer, urethane rubber etc.

0034 If necessary, it is better to add viscosifier into said rubber adhesive. Examples of the viscosifier are lotion resin, polyterpene resin, coumarone Indene resin, petroleum resin, terpenephenol resin, alicyclic hydrocarbon. The concentration of viscosifier in adhesive is not specifically limited. Usually a 20 to 80 weight% of the total weight of adhesive and viscosifier is desired.
It is also preferable to add liquid paraffin, liquid polybutene, liquid polyisoprene, mineral oil lanolin, liquid acrylate etc. into the said rubber adhesive.

0035
Examples of the said water soluble resins are natural polymers of gum arabic, locust bean gum, guar gum, karaya gum, agar gum, starch, carrageenan, Alginic acid, alginate, Propylene glycol alginate, dextran, dextrin, amylase, gelatin, collagen, pullulan, Pectin, amylopectin, chitin, albumen, casein, polyglutamic acid etc; or synthetic polymers of methylcellulose, ethyl cellulose, propyl cellulose, ethyl methyl cellulose, hydroxypropyl cellulose, hydroxyethyl cellulose, hydroxypropylcellulose, hydroxypropyl starch, carboxymethyl starch, alkali metallic carboxymethyl cellulose, alkali metallic cellulose sulfate, cellulose acetate sebacate, starch-acrylic acid grafting copolymer, cross-linked gelatin, phthalic acid modified gelatin, succinic acid modified gelatin etc.; or synthetic polymers of polyvinyl alcohol, polyvinylpyrrolidone, poly(vinyl methyl ester, Methyl vinyl ester, poly acrylic acid, such as polyacrylic acid sodium, carboxyl-vinyl polymer, vinylpyrrolidone-ethyl acrylate copolymer, vinylpyrrolidone-styrene copolymer, vinylpyrrolidone-vinyl acetate copolymer, vinyl acetate-methyl acrylic acid copolymer, vinyl acetate-crotonic acid copolymer, polyvinyl sulfonic acid, polyItaconic acid, polyhydroxyethyl acrylate, polyacrylamide, styrene-maleic acid anhydride copolymer, ethylene-maleic acid anhydride copolymer, acrylamide-acrylic acid copolymer.

0036 If water in cataplasm transdermal preparation is not enough, the drug absorption will be postponed, and a cool feeling will be sensed due to the evaporation of water. However, an overweight of water in cataplasm transdermal preparation can increase the fluidity of the water soluble polymer which makes it difficult to keep the preparation shape. Usually the concentration is 1 to 90% by weight, preferably 5 to 85% by weight.

0037
If necessary, the cataplasm transdermal preparation can be added into resins of acrylic resin, gum, silicon gum, humectants of polyhydric alcohol (for example, glycerin, polypropyleneglycol), inorganic fillers of kaolin, bentonite, lead oxide, titanium dioxide, viscosity regulator and antioxidant etc.

0038 It is desirable to include chemical enhancer(s) into the said base material. Examples of the rtansdermal absorption enhancers are alcohols of menthol, camphorenal, cetyl alcohol etc.; fatty acid esters of isopropyl palmitate, isopropyl myristate etc.; glycerol esters of glyceryl monolaurate, glyceryl monooleate, monocaprin glycerin etc.; acidic amides of lauryl diethanol amide etc.; neutral surfactants of polyethelene glycol dilauryl ether, sodium dodecyl sulfate etc.
A less than necessary addition of chemical enhancer would not obtain the enhancing effect. However, an overdose of the chemical enhancer would reduce the adhesiveness of the matrix. Thus, it is preferable that the base material and chemical enhancer are mixed in a concentration ratio of 100:3 to 40 by weight.

0039 It is also desired to include cholesterol into the said transdermal administration preparation to effectively restrain the skin simulation which caused by the application of the transdermal administration preparation to the skin. For transdermal administration tape preparation, a less than necessary addition of cholesterol would not obtain the effect on skin simulation reducing, while an overdose of cholesterol would reduce the adhesiveness of the adhesive. Thus, it is preferable that the base material and cholesterol are mixed in a concentration ratio of 100:5 to 20 by weight.

0040 It is also desired to include antioxidant into the said transdermal administration preparation to effectively restrain the skin simulation which caused by the application of the transdermal administration preparation to the skin. It is appropriate to mix antioxidant into the preparation. It may also firstly embrocate the ointment which includes antioxidant in base material, then plasters the said bisphosphonate preparation, which can moderate the skin stimulation caused by the said bisphosphonate preparation.
Antioxidant can react with the active oxygen which released by bisphosphonate from skin to moderate the skin stimulation of the preparation.

0041
Examples of antioxidants used in said preparation are sodium nitrite, ascorbic acid, methionine, dibutylhydroxytoluene, soybean lecithin, vitamin E, butylhydroxyanisole, benzotriazole, polyphenol, astaxanthin and tranexamic acid. They can be used alone or combined

0042 The said transdermal administration tape preparation comprises the components mentioned above. It is easy to be plastered on skin for those elderly patients lying in bed. It has highly practical application for its no need to avoid a horizontal position within 30 minutes after taking medication and its improved transdermal absorptivity. Bisphosphonate and its derivatives can be delivered safely and quickly for a prolonged time to maintain a therapeutic plasma concentration, which is an effective cure for bone calcium metabolic diseases, such as high calcium in blood which resulted from osteoporosis, osteitis deformans and Malignant tumors.

0043

### Examples of adhesive copolymerization.

### Adhesive A

0.3g of the mixture of potassium peroxisulfate and potassium pirosulfide in a ratio of 2:1 by weight was dissolved in 30ml water to form a catalyzer solution. A blend solvent of water and acetonitrile in a ratio of 1:1 by volume, 500g acrylic acid, 60g 2-ethylhexyl acrylate and 60 N-vinyl-2-pyrrolidone were added into a 3000ml reactor and mixed using the stirrer attached to the reactor. Then the solution was heated to 75 degree C in a nitrogen atmosphere and the said catalyzer solution was added to startup the copolymerization. 2 hours later, 4ml of the catalyzer solution was added. The copolymerization was carried on at 70 degree C for 10 hours to produce a solution of adhesive A with a solid content of 25% by weight.

0044

### Adhesive B

A cross-linking reaction was proceeded by adding 0.25g aluminum acetylacetonate in THF into 100g of said adhesive A to produce adhesive B.

### Adhesive C

300g ethyl acetate, 75g 2-ethylhexyl acrylate, 25g N-vinyl-2-pyrrolidone and 0.005g azobisisbutyronitrile solution were added into a 500ml flask and stirred. The solution was heated to 75 degree C for 15 hours in a nitrogen atmosphere to produce a solution of adhesive C with a solid content of 25% by weight.

0045

### Adhesive D

300g ethyl acetate, 95g 2-ethylhexyl acrylate, 95g 2-ethylhexyl acrylate, 5g acrylic acid and 0.005g azobisisbutyronitrile were added into a 500ml flask and stirred. The solution was heated to 75 degree C. for 15 hours in a nitrogen atmosphere to produce a solution of adhesive D with a solid content of 25% by weight.

0046

### Synthesis of PEG modified alendronate

A reaction between 1g of alendronate and 100mg of polyetyleneglycol (PEG, average mass molecule of 5200, Japan Fat & Oil Company made) activated against amino group was carried out in 20ml of phosphate buffer solution of pH 9.2 at 4 degree C in dark place. The product was dialyzed and freezing dried. A PEG modified alendronate was obtained.

0047

### Example 1 to example 10, example 12

Given amount of alendronate, incadronate, PEG modified alendronate as shown in table 1 were dissolved in water of 20 times weight to form a water solution. Given amount of adhesive solution A to D, glycerin, cholesterol, sodium dodecyl sulfate and monocaprin glycerin ester, as shown in table 1 were mixed and added with the said water solution, thus resulting in a coating solution. The coating solution was then applied to a urethane film and dried to form an adhesive base layer with a thickness of 200µm to resulting in the desired transdermal administration type pharmaceutical preparation in tape form.

0048

### Example 11

32 g copolymer of ethylene-isoprene-styrene (Kraton Polymers Japan Ltd, trade name: Kraton D1107), 40g alicyclic hydrocarbon (Arakawa Chemical Industries,Ltd., trade name: Alcon P-90) and 28g liquid paraffin (Nacalai Co. Ltd.) were mixed in kneader at 120 degree C to produce hot melted adhesive E. When the temperature of the kneader fell to 90 degree C, 2g sodium dodecyl sulfate and 10g alendronate were added and mixed to produce a coating solution. The coating solution was then applied to a 40 µm thick PET film and dried to form an adhesive base layer with a thickness of 200µm to resulting in the desired transdermal administration type pharmaceutical preparation in tape form.

0049

**Table 1**

| Example | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Adhesive A | - | - | - | - | 100 | 100 | 100 | 100 | - | - | - | - | 100 | |
| Adhesive B | 100 | 100 | 100 | 100 | - | - | - | - | - | - | - | 100 | | 100 |
| Adhesive C | - | - | - | - | - | - | - | - | 100 | - | - | - | | |
| Adhesive D | - | - | - | - | - | - | - | - | - | 100 | - | - | | |
| Adhesive E | - | - | - | - | - | - | - | - | - | - | 100 | - | | |
| glycerin | 200 | 200 | - | 200 | - | - | - | - | 200 | 200 | - | 200 | | 200 |
| cholesterol | - | 10 | 10 | - | - | - | 10 | - | 10 | 10 | - | - | | |
| sodium dodecyl sulfate | - | 2 | 3 | 3 | 3 | - | - | - | 2 | 2 | 2 | 2 | 3 | |
| monocaprin glycerin ester | 5 | - | 7 | 5 | 5 | - | - | - | 5 | 5 | - | - | 5 | 5 |
| alendronate | 10 | 10 | - | - | 10 | 10 | - | - | 10 | 10 | 10 | - | 10 | 10 |
| incadronate | - | - | 10 | 10 | - | - | 10 | 10 | - | - | - | - | | |
| PEG modified alendronate | - | - | - | - | - | - | - | - | - | - | - | 40 | | |
| dibutylhydr oxytoluene | | | | | | | | | | | | | 2 | |
| Vitamin E | | | | | | | | | | | | | | 5 |

### Weight of adhesives refers to solid content

0050

### Example 13, 14

Given amount of adhesive A or B solution, glycerin or sodium dodecyl sulfate as shown in table 1 was mixed to form a adhesive solution. Given amount of alendronate was dissolved in water of 20 times weight and added into the said adhesive solution. Then given amount of dibutylhydroxytoluene or vitamin E as shown in table 1 was add to form a coating solution. The coating solution was then applied to a 40 µm thick urethane film and dried to form an adhesive base layer with a thickness of 200µm to resulting in the desired transdermal administration type pharmaceutical preparation in tape form.

0051

The transdermal absorptitivity of said transdermal administration type pharmaceutical preparation in tape was examined and the results of which are shown in Table 2.

### Skin permeation experiment

The human skin was sandwiched between the donor and receptor compartments of improved Franze diffusion cell. The pharmaceutical preparation obtained above was mounted on the skin on the donor side. The receptor cell was filled with PBS buffer solution (pH 7.4). Cells were placed on a magnetic stirring plate with water bath maintained at 37°C. At the time intervals of 4, 8 and 24 hours, receptor solution was taken from the receptor compartment and samples were analyzed by HPLC method to calculate the accumulated permeation amount as indicated in Table 2.

0052 The skin irritation, adhesiveness and skin remain of the adhesive of the pharmaceutical preparation obtained above were examined, the results of which are shown in Table 2.

### skin irritation test

The pharmaceutical preparation obtained above was applied to upper arms of four volunteers with the size of 3.14cm² in area (diameter of 2cm in a cycle) for 24 hours. Jus after the removal of the patch, the irritation reaction was assessed by naked eyes and using a 5-point scale of 0-4 as follows; 0 = No erythema; 1 = Very slight erythema; 2 = Well defined rythema; 3 = moderate erythema; 4 = Strong erythema to eschar formation.

0053

### Skin adherence and skin remaining evaluation

The pharmaceutical preparation obtained above was applied to upper arms of four volunteers with the size of 3.14cm² in area (diameter of 2cm in a cycle) for 24 hours. Just after the removal of patch, patch adherence and residual adhesive on the skin were estimated by naked eyes.

0054
Results in table 2 indicated that the transdermal administration type preparation in tape form has better transdermal absorptivity and less skin stimulation when PEG modified alendronate was used. It is safe and practical for a prolonged time usage.

0055

**Table 2**

| | Accumulated amount (µg/cm²) | | | Skin stimulation | Skin adherence | adhesive residual on skin |
|---|---|---|---|---|---|---|
| | 4 hours | 8 hours | 24 hours | | | |
| Example1 | 0.3 | 5.2 | 14.3 | 2.75 | Good | No residual |
| Example2 | 0.4 | 6.5 | 16.1 | 1.00 | Good | No residual |
| Example3 | 0.5 | 7.8 | 21.0 | 0.75 | Good | No residual |
| Example4 | 0.4 | 8.3 | 24.8 | 2.50 | Good | No residual |
| Example 5 | 0.3 | 7.9 | 25.6 | 2.75 | Good | No residual |
| Example 6 | 0.0 | 2.9 | 9.1 | 0.50 | Good | No residual |
| Example 7 | 0.2 | 3.6 | 9.5 | 2.75 | Good | No residual |
| Example 8 | 0.0 | 3.4 | 8.8 | 3.00 | Good | No residual |
| Example 9 | 0.6 | 8.6 | 27.0 | 0.50 | Good | No residual |
| Example 10 | 0.5 | 7.6 | 29.3 | 0.75 | Good | No residual |
| Example 11 | 0.1 | 4.3 | 11.2 | 3.00 | Good | No residual |
| Example 12 | - | - | - | 0.00 | Good | No residual |
| Example 13 | 0.3 | 7.5 | 23.9 | 0.25 | Good | No residual |
| Example 14 | 0.4 | 5.8 | 16.2 | 0.25 | Good | No residual |

## Claims

1. A pharmaceutical preparation in tape form comprising
(1) a backing,
(2) an adhesive base layer formed on said backing; said adhesive base layer consisting essentially of
(i) active ingredient of bisphosphonates or their derivatives of medically effective amount
(ii) an adhesive base material of acrylic copolymer

2. A pharmaceutical preparation according to claim 1, wherein said bisphosphonates include their derivatives which were synthesized by the reaction between amino group of bisphosphonate and polyethyleneglycol (PEG), amino acid, or carboxylic acid etc.

3. A pharmaceutical preparation in tape according to claim 4, wherein said adhesive base is consisting of
(1) medically effective amount of bisphosphonates
(2) an adhesive base material of acrylic copolymer which is copolymerized of
(a) acrylic acid
(b) 2-ethylhexyl acrylate
(c) polar monomer (except for acrylic acid)
wherein the concentration ratio of (a), (b) and (c) are 50 to 88%,: 8 to 40%, : 1 to 40%, by weight respectively.
(3) polyhydric alcohol
wherein the concentration ratio of (2) and (3) are 100 : 150 to 600 by weight.

4. A pharmaceutical preparation according to claim 5, wherein said polar monomer is selected from group of N-vinyl-2-pyrollidone, vinyl acetate, methoxyethyl acrylate, 2-hydroxyehyl acrylate etc.

5. A pharmaceutical preparation according to claim 5 or 6, wherein said polyhydric alcohol is at least one selected from group of glycerin, diglycerin, propylene glycol, butylenes glycol, polyethylene glycol and polypropylene glycol etc.

6. A pharmaceutical preparation according to claim 5,6 or 7, wherein said acrylic copolymer is cross-linked using at lease one metal chelate cross-link agent selected from group of aluminum acetylacetonate, aluminium hydrate gel and aluminium glycinate etc.

7. A pharmaceutical preparation according to claim 1∼6 which containing cholesterol.

8. A pharmaceutical preparation according to claim 1∼7 which containing antioxidant.

9. A pharmaceutical preparation according to claim 8, wherein said antioxidant is at least one selected from the group of sodium nitrite, ascorbic acid, methionine, dibutylhydroxytoluene, soybean lecithin, vitamin E, butylhydroxyanisole, benzotriazole, polyphenol, astaxanthin and tranexamic acid.
